# EUROPEAN PATENT SPECIFICATION

(11) **EP 1 472 999 B1**
(45) Date of publication and mention of the grant of the patent: **31.03.2010**
(21) Application number: 04101775.7
(22) Date of filing: 28.04.2004
(51) Int. Cl.: A61F 2/40

(54) **Prosthesis for the articulation of the shoulder**
Prothese für das Schultergelenk.
Prothèse pour l'articulation de l'épaule.

(30) Priority: 30.04.2003 IT UD20030092
(43) Date of publication of application: 03.11.2004
(73) Proprietor: LIMA Lto SpA, 33030 Villanova di San Daniele del Friuli (UD) (IT)
(72) Inventor: Dalla Pria, Paolo, 33100 Udine (IT)
(74) Representative: Petraz, Gilberto Luigi

(56) References cited:
- EP-A- 0 299 889
- FR-A- 2 579 454
- FR-A- 2 652 498
- FR-A- 2 699 400
- US-B1- 6 197 063

## Description

### FIELD OF THE INVENTION

The present invention concerns a prosthesis for the articulation of the shoulder. To be more exact, the prosthesis of the shoulder according to the invention can be configured both as a normal prosthesis wherein a substantially semi-spherical head is associated with the humerus and articulates in a mating concave seating, natural or artificial, associated with the glenoid cavity, and also as an inverse prosthesis, wherein a substantially spherical head is associated with the glenoid cavity and articulates in an artificial concave seating associated with the humerus. FR-A-2652498 disclosed a prosthesis for a shoulder having the features of the preamble of the main claim.

### BACKGROUND OF TEE INVENTION

Prostheses for the articulation of a shoulder are substantially of two types, the prostheses which reproduce the normal gleno-humeral anatomy, and the so-called inverse prostheses.

The former, which are called total when they also include an artificial hollow seating attached to a glenoid cavity, comprise as essential elements a hemispherical humeral head and a shaft implanted into the humerus on which said humeral head is directly assembled.

On the contrary, inverse prostheses comprise a concave humeral cup, also assembled directly on the shaft implanted in the humerus, which defines the articulation seating for a spherical body attached to the glenoid cavity.

Usually, inverse prostheses are used instead of normal prostheses when there are serious muscular degenerations of the shoulder, particularly of the muscles of the rotator cuffs. The degeneration of such muscles causes a prevalent action by the deltoid, which tends to draw the humerus upwards, consequently making the humeral head, or bone head, or head of the normal prosthesis move upwards, with the risk of making it knock against the protrusion which prolongs the spine of the scapula, known as the acromion.

In order to pass from a normal configuration to an inverse configuration of the prosthesis, during the operation, the humeral head is removed from the implant shaft, where it is possible, that is, when the two elements are made in different pieces, and it is replaced with a concave humeral cup. Similarly, the hollow artificial seating is removed from the glenoid cavity and is replaced by the aforementioned spherical body.

Consequently the shaft remains implanted in the humerus, and only the articulation elements are replaced.

Prostheses of a known type have the disadvantage, however, that when passing from one configuration to the other it is not possible to choose and correct the position of the replacement articulation element, such as the humeral cup or the head, in the desired manner. This is due to the fact that the fixed position of the implant shaft inevitably constrains the position, such as the height and orientation, of the articulation element which is assembled thereon.

For example, in the case of traumatic events, passing from a normal to an inverse configuration, it would normally be better to have the height at which the cup is assembled with respect to the implant shaft different from that of the humeral head, but this is not possible during the replacement step, since the position of the shaft defines a univocal assembly configuration that cannot be modified.

One purpose of the invention is to achieve a prosthesis for the articulation of the shoulder which can be configured both as a normal prosthesis and also as an inverse prosthesis, and which, during the passage from one configuration to the other, allows to select the position of the humeral articulation element, which is assembled in replacement, in order to optimize the overall configuration of the prosthesis according to the anatomical-pathological conditions of the shoulder and the different type of prosthesis installed.

To be more exact, the purpose of the present invention is to make possible to adjust the position and hence the height, the orientation, the inclination or other, of the replacement humeral articulation element, while keeping a substantial part of the element that attaches to the humerus fixed.

The Applicant has devised, tested and embodied the present invention to achieve this purpose and obtain other advantages, and to overcome the shortcomings of the state of the art.

### SUMMARY OF THE INVENTION

The present invention is set forth and characterized essentially in the main claim, while the dependent claims describe other innovative characteristics of the invention.

A prosthesis of the shoulder for the articulation of a humerus with respect to a scapula comprises at least a first anchorage or humeral element, able to be attached to the humerus, and at least a second anchorage or glenoid element, able to be attached to said glenoid cavity. The first anchorage element is able to selectively support an articulation element of the concave type, or the convex type, in order to cooperate with a mating convex or concave articulation element associated with the glenoid cavity. According to the invention, the prosthesis comprises at least a replaceable adaptor element, located between the humeral anchorage element and the relative articulation element, concave or convex, which allows to correct the assembly position of the latter, when an articulation element of the concave type is replaced by an articulation element of the convex type, or vice versa.

In one embodiment of the invention, the prosthesis comprises at least an adaptor element of a first type which, on one side, has means to couple with the humeral anchorage element and, on the other side, a substantially concave portion which defines the housing seating for the articulation element of the concave type. The prosthesis also comprises an adaptor element of a second type provided with implant means to attach a convex humeral articulation element.

In this way, when the prosthesis is transformed from a normal configuration to an inverse configuration, or vice versa, the relative adaptor element is also removed; when a new implant is made, it is possible to choose the most suitable adaptor element, that is, the one which allows to position the relative articulation element at an optimum height, and with an optimum orientation, with respect to the mating articulation element associated with the glenoid cavity, also according to the anatomical-pathological characteristics of the patient.

### BRIEF DESCRIPTION OF THE DRAWINGS

These and other characteristics of the present invention will become apparent from the following description of a preferential form of embodiment, given as a non-restrictive example with reference to the attached drawings wherein:
- fig. 1 shows a section of a prosthesis for the shoulder according to the present invention in a first normal configuration;
- fig. 2 shows a section of the prosthesis in fig. 1 in a second inverse configuration;
- fig. 3 shows an exploded detail of the prosthesis in fig. 1;
- fig. 4 shows an exploded detail of the prosthesis in fig. 2.

### DETAILED DESCRIPTION OF A PREFERENTIAL FORM OF EMBODIMENT OF THE INVENTION

With reference to the attached drawings, the reference number 10 denotes a prosthesis according to the present invention able to be implanted in a shoulder in order to allow the articulation of a humerus 12 in a relative glenoid cavity 15 of a scapula 54, only partly shown.

The prosthesis 10 comprises a shaft 38, made of metal, to anchor in the humerus 12, and an anchorage element 14 to anchor to the glenoid 15, or glenoid element, made of metal or plastic. The humeral shaft 38 is inserted in depth into the humerus 12 by means of percussion or screwing, so as to remain permanently fixed in the humerus 12.

According to a variant, the humeral shaft 38 can be at least partly cemented to the bone.

In order to increase the stability and duration of the implant, in a variant embodiment the shaft 38 has a transverse section with a triple or double taper facing towards the inner end.

According to other embodiments, the taper can be single or with a different value of multiplicity, for example quadruple.

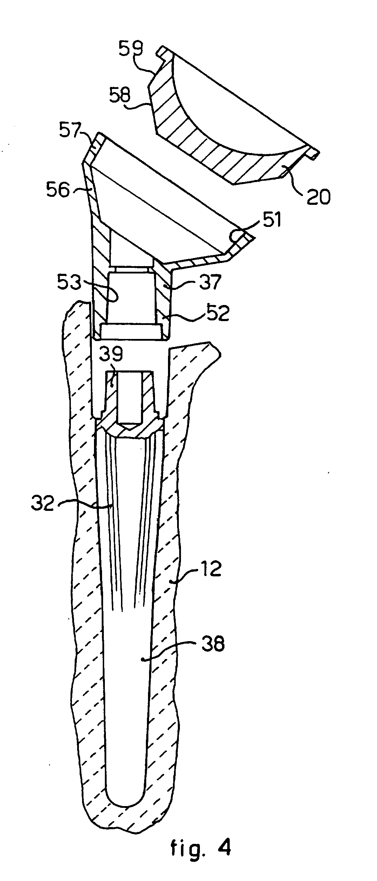

Moreover, the outer surface of the shaft 38 comprises a plurality of longitudinal fins 32, in this case only in the upper portion (fig. 4), which increase the interference, and hence the gripping capacity, of the shaft 38 in the mating anchorage hole made in the bone wall of the humerus 12. According to a variant, such fins 32 are replaced by or integrated with screwing threads, spiral segments, or other.

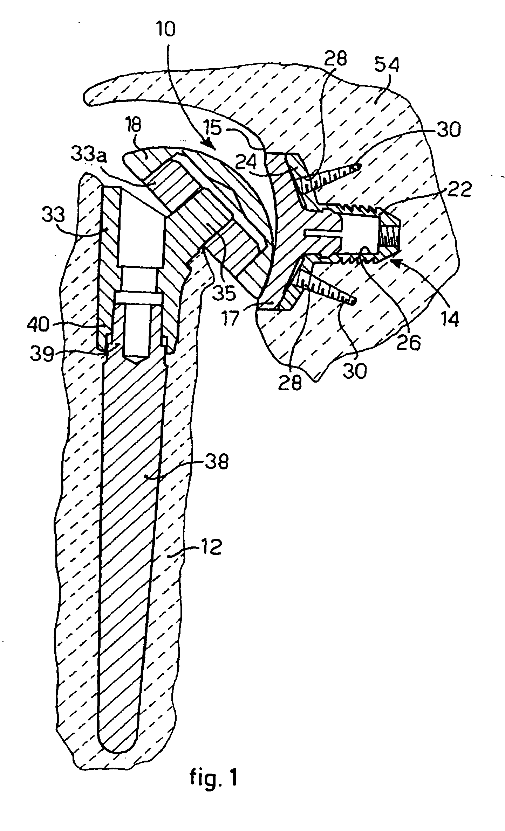

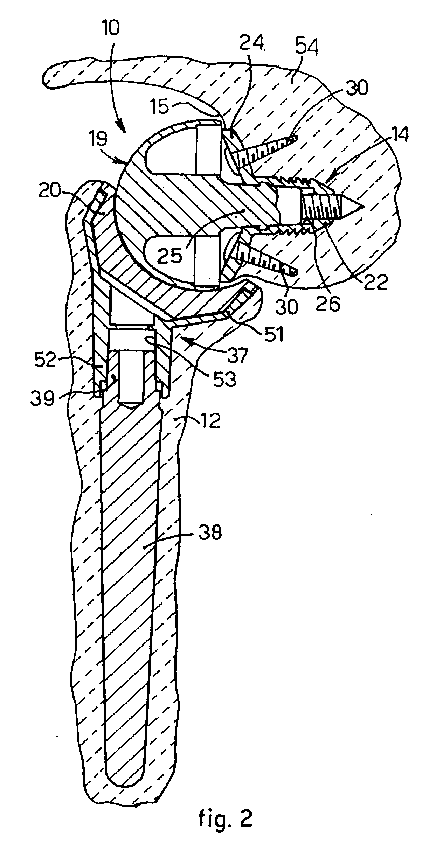

The anchorage element 14 (figs. 1 and 2) comprises an implant shaft 22 and a plate 24 and is also provided with a through central hole 26. A plurality of holes 28 are made laterally with respect to the hole 26 for the insertion of attachment screws 30 that attach the anchorage element 14 to the wall of the bone seating.

The anchorage element 14 is implanted by screwing or cementing the implant shaft 22 into a mating seating made in the glenoid 15, and the attachment screws 30 are subsequently screwed in.

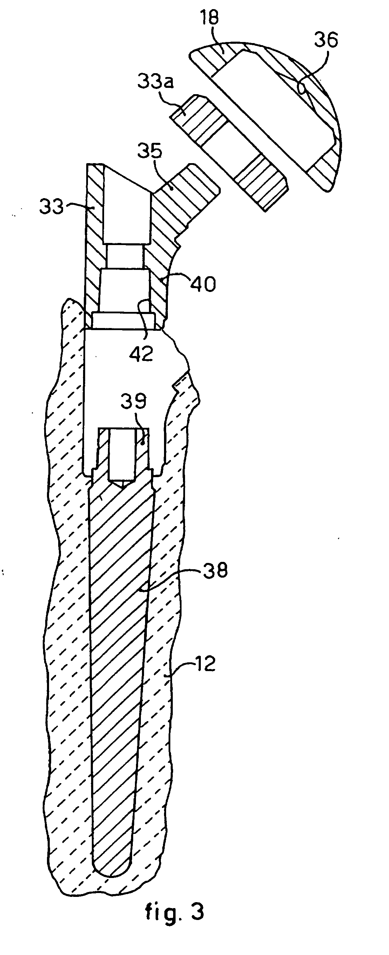

The prosthesis 10 can be configured both as a normal prosthesis (figs. 1 and 3) and also as an inverse prosthesis (figs. 2 and 4), sharing both the anchorage element 14 that anchors the prosthesis 10 to the glenoid 15 of the scapula 54, and also the humeral shaft 38.

In the case of a normal configuration, a hemi-spherical humeral head 18 (figs. 1 and 3), in this case hollow inside, is attached to the humeral shaft 38; the hemispherical humeral head 18 is able to articulate with an insert 17, which is applied solidly on the plate 24 of the anchorage element 14.

In the case of an inverse configuration, instead of the humeral head 18 mentioned above, a humeral cup 20 (figs. 2 and 4) is attached to the humeral shaft 38 and allows the articulation of a spherical body called the glenoid head 19, assembled on the anchorage element 14 by means of an implant pin 25.

According to the present invention, in order to attach and position, respectively, the hemi-spherical humeral head 18 or the humeral cup 20 to the shaft 38, the prosthesis 10 comprises a first adaptor body 33 (figs. 1 and 3) and a second adaptor body 37 (figs. 2 and 4), which are able to be assembled on the top 39 of the humeral shaft 38.

In this case, the first adaptor body 33 is substantially cylindrical in shape and comprises a lower portion 40, which has a hollow seating 42 inside, where the top 39 of the shaft 38 (fig. 3), substantially conical in shape, is inserted and fixed by means of conical coupling.

The first adaptor body 33 also comprises, so that it can be attached to the humeral head 18, an implant pin 35, which is arranged oblique with respect to the main axis of the adaptor body 33, and is able to be implanted in a conical element 33a, arranged in a mating inner cavity 36 of the humeral head 18.

The second adaptor body 37 comprises an upper portion 51 (figs. 2 and 4) with a concave shape to house the humeral cup 20, and a lower portion 52, inside which a hollow seating 53 is made, substantially equal to the hollow seating 42 of the first adaptor body 33, to allow coupling with the top 39 of the shaft 38.

In the embodiment shown here, to allow a stable coupling with the humeral cup 20, the inner surface of the upper portion 51 has a conical portion 57 which couples with a mating conical portion 59 of the outer surface of the humeral cup 20. The conical portions 57 and 59 extend respectively with conical surfaces 56 and 58 which, during the implant step, are inserted one inside the other.

When it is necessary to replace a normal prosthesis by an inverse prosthesis, the hemi-spherical humeral head 18, the relative adaptor body 33 and the conical element 33a are removed, and are replaced by the aforesaid humeral cup 20 and the relative adaptor body 37.

At the same time, on the side of the glenoid cavity 15, it is necessary to remove the insert 17 from the plate 24, and insert the glenoid head 19.

In this way there is a double advantage: when passing from the normal to the inverse configuration, it is not necessary either to remove and replace the humeral shaft 38, nor to remove the glenoid anchorage element 14; moreover, it is possible, by replacing the first adaptor body 33 by the second adaptor body 37, to correct the position of the humeral head 18 or of the humeral cup 20 with respect to the mating glenoid articulation element, according to the conditions of the humerus and/or its degenerative state, and in general with respect to the anatomical-pathological conditions of the patient.

To be more exact, the fact that the humeral component of the prosthesis 10 is made in several parts allows to choose between a plurality of adaptor bodies 33, 37, having for example a greater or lesser length, a different shape, a more or less accentuated inclination of the pin 35, or a more or less deep housing seating of the upper portion 51. These options allow to vary the height, the position, or the orientation of the relative articulation elements 20, 18.

It is clear, however, that modifications and/or additions of parts may be made to the universal prosthesis 10 as described heretofore, without departing from the field and scope of the present invention.

## Claims

1. Prosthesis of the shoulder for the articulation of a humerus (12) in a scapula (54), comprising a first anchorage element (38) able to be attached to the humerus (12), a second anchorage element (14) able to be attached to a glenoid cavity (15) of said scapula (54), an articulation element of the concave type (20), and an articulation element of the convex type (18) which said first anchorage element (38) is able to selectively support, a mating convex articulation element (19) and a mating concave articulating element (17), associated with the glenoid cavity (15), able to cooperate with said articulation elements of the concave and convex types, said second anchorage element (14) including means (26) for selectively attaching either said concave mating articulation element (17) able to articulate with said convex type articulation element (18) mounted on said first anchorage element (38) or said convex mating articulation element (19) able to articulate with said concave type articulation element (20) mounted on said first anchorage element (38) in substitution of said convex mating articulation element (18), **characterised in that** it further comprises at least an adaptor element of a first type (33), provided on one side with coupling means (42) for attachment to said first anchorage element (38), and, on the other side, with implant means (35) for attachment to said articulation element of the convex type (18), and an adaptor element of a second type (37) provided on one side with coupling means (53) for coupling with said first anchorage element (38) and, on the other side, having a housing seating (51) for said articulation element of the concave type (20).

2. Prosthesis as in claim 1, **characterized in that** said adaptor element (33, 37) is located between said first anchorage element (38) and the relative articulation element (20, 18), and is able to allow the adjustment of the assembly position when an articulation element of the concave type (20) is replaced by an articulation element of the convex type (18), or vice versa.

3. Prosthesis as in claim 2. **characterized in that** said implant means (35) are able to be coupled with said articulation element of the convex type (18) by means of conical coupling.

4. Prosthesis as in claim 2 or 3, **characterized in that** said articulation element of the convex type (18) has a cavity (36) which houses a mating coupling element (33a), and **in that** said implant means (35) are able to be implanted in said mating coupling element (33a).

5. Prosthesis as in any claim from 2 to 4 inclusive, **characterized in that** said implant means (35) are arranged in an inclined position with respect to the main axis of said adaptor element of the first type (33).

6. Prosthesis as in any claim from 2 to 5 inclusive, **characterized in that** the surface of said housing seating (51) has contiguous conical portions (57) able to be coupled with mating contiguous conical portions (59) made on the outer surface of said articulation element of the concave type (20).

7. Prosthesis as in any claim hereinbefore, **characterized in that** said replaceable adaptor element (33, 37) is able to be coupled with said humeral anchorage element (38) by means of conical coupling.

8. Prosthesis as in any claim hereinbefore, **characterized in that** said humeral anchorage element comprises a shaft (38) including a longitudinal transverse section with a single or multiple taper facing towards its inner end.

## Patentansprüche

1. Schulterprothese für die Artikulation eines Humerus (12) in einer Skapula (54), umfassend ein erstes am Humerus (12) befestigbares Verankerungselement (38), ein zweites an einer Gelenkpfanne (15) der Skapula (54) befestigbares Verankerungselement (14), ein Artikulationselement des konkaven Typs (20) und ein Artikulationselement des konvexen Typs (18), wobei das erste Verankerungselement (38) dazu fähig ist, ein zusammenpassendes konvexes Artikulationselement (19) und ein zusammenpassendes konkaves Artikulationselement (17), das der Gelenkpfanne (15) zugeordnet ist, selektiv abzustützen, das dazu fähig ist, mit den Artikulationselementen des konkaven und konvexen Typs zusammenzuarbeiten, wobei das zweite Verankerungselement (14) ein Mittel (26) aufweist zum selektiven Befestigen entweder des konkaven zusammenpassenden Artikulationselements (17), das dazu fähig ist, mit dem Artikulationselement des konvexen Typs (18) artikuliert zu werden, das am ersten Verankerungselement (38) angebracht ist, oder des konvexen zusammenpassenden Artikulationselements (19), das dazu fähig ist, mit dem Artikulationselement des konkaven Typs (20) artikuliert zu werden, das am ersten Verankerungselement (38) angebracht ist, als Ersatz für das konvexe zusammenpassende Artikulationselement (18), **dadurch gekennzeichnet, dass** sie ferner mindestens ein Adapterelement eines ersten Typs (33), das auf der einen Seite mit Kopplungsmitteln (42) zur Befestigung des ersten Verankerungselements (38), und auf der anderen Seite mit Implantatsmitteln (35) zum Befestigung an dem Artikulationselement des konvexen Typs (18) versehen ist, sowie ein Adapterelement eines zweiten Typs (37) umfasst, das auf der einen Seite mit Kopplungsmitteln (53) zur Kopplung mit dem ersten Verankerungselement (38) versehen ist und auf der anderen Seite eine Gehäuselagerung (51) für das Artikulationselement des konkaven Typs (20) aufweist.

2. Prothese nach Anspruch 1, **dadurch gekennzeichnet, dass** das Adapterelement (33, 37) zwischen dem ersten Verankerungselement (38) und dem relativen Artikulationselement (20, 18) angeordnet und dazu fähig ist, die Einstellung der Zusammenbauposition zu gestatten, wenn ein Artikulationselement des konkaven Typs (20) durch ein Artikulationselement des konvexen Typs (18) ersetzt wird oder umgekehrt.

3. Prothese nach Anspruch 2, **dadurch gekennzeichnet, dass** die Implantatsmittel (35) dazu fähig sind, mittels einer kegeligen Kopplung mit dem Artikulationselement des konvexen Typs (18) gekoppelt zu werden.

4. Prothese nach Anspruch 2 oder 3, **dadurch gekennzeichnet, dass** das Artikulationselement des konvexen Typs (18) einen Hohlraum (36) hat, in dem ein zusammenpassendes Kopplungselement (33a) untergebracht ist, und dass die Implantatsmittel (35) dazu fähig sind, in dem zusammenpassenden Kopplungselement (33a) implantiert zu werden.

5. Prothese nach einem der Ansprüche 2 bis einschließlich 4, **dadurch gekennzeichnet, dass** die Implantatsmittel (35) in einer geneigten Position bezüglich der Hauptachse des Adapterelements des ersten Typs (33) angeordnet sind.

6. Prothese nach einem der Ansprüche 2 bis einschließlich 5, **dadurch gekennzeichnet, dass** die Oberfläche der Gehäuselagerung (51) angeformte kegelige Teile (57) hat, die dazu fähig sind, mit zusammenpassenden angeformten kegeligen Teilen (59), die auf der Außenoberfläche des Artikulationselements des konkaven Typs (20) ausgebildet sind, gekoppelt zu werden.

7. Prothese nach einem der bisherigen Ansprüche, **dadurch gekennzeichnet, dass** das ersetzbare Adapterelement (33, 37) dazu fähig ist, mit dem Humerus-Verankerungselement (38) mittels einer kegeligen Kopplung gekoppelt zu werden.

8. Prothese nach einem der bisherigen Ansprüche, **dadurch gekennzeichnet, dass** das Humerus-Verankerungselement einen Schaft (38) aufweist, der einen längs verlaufenden Querschnitt mit einem einzigen oder mit mehrfachen Kegeln aufweist, die zu seinem inneren Ende hin weisen.

## Revendications

1. Prothèse de l'épaule pour l'articulation d'un humérus (12) dans une omoplate (54), comprenant un premier élément d'ancrage (38) pouvant être fixé sur l'humérus (12), un second élément d'ancrage (14) pouvant être fixé sur une cavité glénoïde (15) de ladite omoplate (54), un élément d'articulation de type concave (20) et un élément d'articulation de type convexe (18), lequel premier élément d'ancrage (38) peut supporter de manière sélective un élément d'articulation convexe correspondant (19) et un élément d'articulation concave correspondant (17) associé avec la cavité glénoïde (15) pouvant coopérer avec lesdits éléments d'articulation de types concave et convexe, ledit second élément d'ancrage (14) comprenant des moyens (26) pour fixer sélectivement ledit élément d'articulation concave correspondant (17) pouvant s'articuler avec ledit élément d'articulation de type convexe (18) monté sur ledit premier élément d'ancrage (38) ou ledit élément d'articulation correspondant convexe (19) pouvant s'articuler avec ledit élément d'articulation de type concave (20) monté sur ledit premier élément d'ancrage (38) en substitution dudit élément d'articulation correspondant convexe (18), **caractérisée en ce qu'**elle comprend en outre au moins un élément d'adaptateur d'un premier type (33), prévu sur un côté avec des moyens de couplage (42) pour la fixation sur ledit premier élément d'ancrage (38), et de l'autre côté, avec des moyens d'implant (35) pour la fixation sur ledit élément d'articulation de type convexe (18) et un élément d'adaptateur d'un second type (37) prévu sur un côté avec des moyens de couplage (53) pour se coupler avec ledit premier élément d'ancrage (38) et, de l'autre côté, ayant un siège de boîtier (51) pour ledit élément d'articulation du type concave (20).

2. Prothèse selon la revendication 1, **caractérisée en ce que** ledit élément d'adaptateur (33, 37) est positionné entre ledit premier élément d'ancrage (38) et l'élément d'articulation (20, 18) relatif, et peut permettre l'ajustement de la position d'assemblage lorsqu'un élément d'articulation du type concave (20) est remplacé par un élément d'articulation du type convexe (18) ou vice versa.

3. Prothèse selon la revendication 2, **caractérisée en ce que** lesdits moyens d'implant (35) peuvent être couplés avec ledit élément d'articulation du type convexe (18) au moyen du couplage conique.

4. Prothèse selon la revendication 2 ou 3, **caractérisée en ce que** ledit élément d'articulation du type convexe (18) a une cavité (36) qui loge un élément de couplage correspondant (33a) et **en ce que** lesdits moyens d'implant (35) peuvent être implantés dans ledit élément de couplage de raccordement (33a).

5. Prothèse selon l'une quelconque des revendications 2 à 4 y compris, **caractérisée en ce que** lesdits moyens d'implant (35) sont agencés dans une position inclinée par rapport à l'axe principal dudit élément d'adaptateur du premier type (33).

6. Prothèse selon l'une quelconque des revendications 2 à 5 y compris, **caractérisée en ce que** la surface dudit siège de boîtier (51) a des parties coniques contiguës (57) pouvant être couplées avec des parties coniques contiguës correspondantes (59) réalisées sur la surface externe dudit élément d'articulation du type concave (20).

7. Prothèse selon l'une quelconque des revendications précédentes, **caractérisée en ce que** ledit élément d'adaptateur remplaçable (33, 37) peut être couplé avec ledit élément d'ancrage huméral (38) au moyen du couplage conique.

8. Prothèse selon l'une quelconque des revendications précédentes, **caractérisée en ce que** ledit élément d'ancrage huméral comprend une tige (38) comprenant une section transversale longitudinale avec un cône unique ou plusieurs cônes orientés vers son extrémité interne.
